# EUROPEAN PATENT APPLICATION

(11) **EP 1 308 092 A1**
(43) Date of publication of application: **07.05.2003**
(21) Application number: 00953424.9
(22) Date of filing: 09.08.2000
(51) Int. Cl.: A01K 67/027, C12N 15/09

(54) **METHOD OF CONSTRUCTING GENE MANIPULATION ANIMAL AND CLONE ANIMAL**

(71) Applicant: CENTRAL INSTITUTE FOR EXPERIMENTAL ANIMALS, Kawasaki-shi, Kanagawa 216-0001 (JP)
(72) Inventor: KONO, Tomohiro, Tokyo 115-0042 (JP); ONO, Yukiko, Ashigarakami-gun, Kanagawa 258-0021 (JP); SHIMOZAWA, Nobuhiro, Domiru-kashima 103, Kawasaki-shi, Kanagawa 211-0041 (JP)
(74) Representative: Maschio, Antonio
(86) International application number: JP0005326
(87) International publication number: WO02011529

(57) **Abstract**

A genetically engineered cloned animal is created by synchronizing the cell cycle of an embryonic stem cell on which genetic engineering has been performed to metaphase, transplanting the nucleus of this cell to the cytoplasm of an enucleated unfertilized ovum, and activating the transplanted nucleus. An animal created by this method is useful as a model animal for various diseases.

## Description

### Technical field of the invention

The present invention relates to a method of creating genetically engineered animals and a method of creating cloned animals (a group of genetically identical animals). Animals created by these methods are useful as model animals for various diseases, etc.

### Background Art

Known methods of creating a genetically engineered animal include a method of injecting a gene into the nucleus of a fertilized ovum and a method of introducing a gene into a embryonic stem cell (ES cell) using homologous recombination. Both of these methods require extensive effort and time. In particular, in case where a gene is introduced into an embryonic stem cell, a desired genetically engineered animal cannot be directly obtained, rather the animal is obtained in the next creation via a germ-line chimeric individual, and so the time and effort that are required is considerable. Further, embryonic stem cells from which germ-line chimera can be efficiently created are limited to specific lines. Thus, when a genetically engineered animal of a line other than the limited lines is sought, repeated backcross to the desired line is necessary.

Moreover, until now, somatic cloned animals have been produced in bovine, sheep, goat, and mouse. These methods of preparing a somatic cloned animal typically use cells whose cell cycle was synchronized to G0/G1 phase by serum deprivation culture, etc., or cells cultured without a synchronization culture. Wakayama T. et al., report on a method using cells synchronized to the metaphase (M phase) (Wakayama T. et al., Proc. Natl. Acad. Sci. USA, 1999, 26, 14984-14989). However, the cells in metaphase that are used in the method are selected using only the size of the cells as an index. Further, in the preparation of these somatic cloned animals, a nuclear transplantation operation is performed only once.

### Problem to be solved by the invention

The present inventors discovered that a somatic clone can be efficiently created, by synchronizing the cell cycle of the donor cells to metaphase and performing a nuclear transplantation twice in a process for producing a somatic clone. This is the subject of an earlier patent application (Japanese Patent Application No. 11-32772, unpublished). However, while this method is a superior method in comparison to conventional methods, there is the problem that many of the neonates die during the process of growing to adulthood.

A first object of the present invention is to solve problems involved in conventional methods of creating a somatic cloned animal, and to provide a means for more efficiently creating a somatic cloned animal,

Further, a second object of the present invention is to solve the above-described problems relating to the creation of a genetically engineered animal, and to provide a means to create a genetically engineered animal in a short period.

### Means for solving the problem

As a result of deliberate study to solve the above problems, the present inventors have made the following findings:
- That it was possible to create a genetically engineered animal in a short period in comparison to conventional methods by creating a somatic clone using the nucleus of an animal cell that had undergone genetic engineering as a donor nucleus.
- By using the nucleus of an embryonic stem cell as a donor nucleus, the rate of mortality in the developmental stage of the created cloned animals can be greatly reduced.

The present invention was completed on the basis of the above findings.

That is, a first aspect of the present invention relates to a method of creating a genetically engineered animal which comprises the following steps:
(1) a step of genetically engineering animal cells;
(2) a step of synchronizing the cell cycle of the genetically engineered animal cells to metaphase;
(3) a step of transplanting the nucleus of a cell whose cell cycle was synchronized to metaphase into cytoplasm of an enucleated unfertilized ovum;
(4) a step of subjecting the transplanted nucleus to activation processing; and
(5) a step of obtaining a neonate derived from the transplanted nucleus.

Further, a second aspect of the present invention relates to a method of creating a genetically engineered animal comprising the following steps:
(1) a step of genetically engineering animal cells;
(2) a step of synchronizing the cell cycle of the genetically engineered animal cells to metaphase;
(3) a step of transplanting the nucleus of a cell whose cell cycle was synchronized to metaphase into cytoplasm of an enucleated unfertilized ovum;
(4) a step of subjecting the transplanted nucleus to activation processing;
(5) a step of transplanting the nucleus that was subject to activation processing into an enucleated fertilized ovum; and
(6) obtaining a neonate derived from the transplanted nucleus.

Further, a third aspect of the present invention relates to a method of creating a cloned animal, which comprises the following steps:
(1) a step of synchronizing the cell cycle of an embryonic stem cell to metaphase;
(2) a step of transplanting the nucleus of the cell whose cell cycle was synchronized to metaphase into cytoplasm of an enucleated unfertilized ovum;
(3) a step of subjecting the transplanted nucleus to activation processing; and
(4) a step of obtaining a neonate derived from the transplanted nucleus.

Further, a fourth aspect of the present invention relates to a method of creating a cloned animal which comprises the following steps:
(1) a step of synchronizing the cell cycle of an embryonic stem cell to metaphase;
(2) a step of transplanting the nucleus of the cell whose cell cycle was synchronized to metaphase into cytoplasm of an enucleated unfertilized ovum;
(3) a step of subjecting the transplanted nucleus to activation processing;
(4) a step of transplanting the nucleus that was subjected to activation processing into a enucleated fertilized ovum; and
(5) a step of obtaining a neonate derived from the transplanted nucleus.

### Disclosure of the Invention

The method of creating a genetically engineered animal according to the present invention comprises the following steps (A) to (D) and step (F), and if necessary, step (E):
(A) a step of genetically engineering animal cells;
(B) a step of synchronizing the cell cycle of the genetically engineered animal cells to metaphase;
(C) a step of transplanting the nucleus of a cell whose cell cycle was synchronized to metaphase to an enucleated unfertilized;
(D) a step of subjecting the transplanted nucleus to activation processing;
(E) a step of transplanting the nucleus that was subjected to activation processing into a enucleated fertilized ovum; and
(F) a step of obtaining a neonate derived from the transplanted nucleus.

In step (A), genetic engineering is performed on an animal cell. The animal cell used here may be any kind of cell, for example, fibroblasts, embryonic stem cells, cumulus oophorus cells, sertoli's cells, etc. can be used. Preferably, embryonic stem cells are used. By using embryonic stem cell, rate of mortality in the developmental stage of the obtained individuals can be reduced. An animal cell is not limited to a particular cell as long as it derives from an animal other than human, and cells originating from mouse, bovine, porcine, sheep, or monkey, etc can be used. Examples of genetic engineering include gene introduction, gene disruption (gene targeting), and gene substitution (knock-in).

In step (B), the cell cycle of the animal cells are synchronized to metaphase. Synchronization to metaphase can be performed with a suitable reagent such as Nocodazole or colcemid, etc.

In step (C), the nucleus of a cell whose cell cycle was synchronized to metaphase is transplanted to the cytoplasm of an enucleated unfertilized ovum. Enucleation of an unfertilized ovum can be performed by, for example, removal by suction of the somewhat transparent portion of the cytoplasm of an unfertilized ovum in a working solution containing cytochalasin B, which is an inhibitor of cytoskeleton polymerization. Transplantation of the nucleus can be performed by cell fusion methods using Sendai virus or electric stimulation or by intracytoplasm injection.

In step (D), activation processing is performed on the nucleus. Examples of activation processing include methods using strontium, ethanol or electrical stimulation etc. A nucleus that has been subjected to activation processing will begin dividing.

In step (E), the nucleus that has been subjected to activation processing is transplanted to an enucleated fertilized ovum. Step (E) is not an essential step, and step (F) may directly follow step (D). Enucleation and transplantation of the nucleus can be performed in a manner similar to step (C). As a fertilized ovum, it is preferable to use one at about 8 to 12 hours after insemination.

In step (F), operations are performed to obtain a neonate derived from the transplanted nucleus. Specifically, operations such as cultivation of the ovum that had been subjected to the activation processing, and transplantation into the uterus of a surrogate mother, etc. are performed. These operations can be performed according to ordinary methods.

A method of creating a genetically engineered animal according to the present invention comprises the following steps (B)' to (D) and step (F), and if necessary, step (E).
(B)' a step of synchronizing the cell cycle of embryonic stem cells to metaphase;
(C) a step of transplanting the nucleus of a cell whose cell cycle was synchronized to metaphase into the cytoplasm of an enucleated unfertilized ovum;
(D) a step of subjecting the transplanted nucleus to activation processing;
(E) a step of transplanting the nucleus that was subjected to activation processing into an enucleated fertilized ovum; and
(F) a step of obtaining a neonate derived from the transplanted nucleus.

Steps (C) to (F) are similar to those of the above method of creating a genetically engineered animal. Further, (B)' is similar to the method of creating a genetically engineered animal, with the exception that it requires an embryonic stem cell as an animal cell.

### Example

Embryonic stem cells, TT2 strain, which had been genetically engineered, were seeded on a feeder cell layer, and allowed to form embryonic stem cell colonies. The TT2 strain which had been genetically engineered was obtained from Yoichi Shinkai, Associate Professor at the Institute for Virus Research, Kyoto University. The G9a gene of this cell is inactivated by homologous recombination by electroporation (Inactivated G9a gene is in hetero form). To obtain feeder cells, a CD-1 mouse fetus having a fetal age of 15.5 days is morcellated with scissors, etc., and fibroblasts obtained by trypsin treatment are cultured and treated with mitomycin. After the embryonic stem cells were sufficiently propagated, they were treated with trypsin-EDTA, and stripped from the culture plate together with the feeder cells, and then seeded in another culture plate. One hour after seeding, the cell suspension in the culture plate was collected. The time taken for embryonic stem cells and feeder cells to adhere to the culture plate differs. Thus, feeder cells adhere to the bottom of the culture plate at 1 hour after seeding but embryonic stem cells remain in a suspended state in the culture solution. Therefore, the suspension of cells collected by the above operation includes only embryonic stem cells. After centrifugation, the cell suspension was suspended in a cryopreservation solution (70% DMEM, 10% DMSO, 20% FBS) at a concentration of 1 x 10⁵ cells/0.5ml per 1 tube and cryopreserved in liquid nitrogen.

The frozen embryonic stem cells were thawed, and 2 x 10⁴ cells were seeded on a culture plate with 60mm diameter that was pre-coated with gelatin, and cultured for 3 days in 15% FBS added ES culture solution. Since this culture plate does not contain feeder cells, no feeder cells are contaminated in the embryonic stem cells from this step onwards. One half of the volume of the culture solution was removed, and the same volume of a culture solution containing Nocodazole was added (final concentration of Nocodazole: 0.4µg/ml) to perform the synchronization process to bring the cells into metaphase. Since damage to cells was observed when time for Nocodazole treatment was too long, processing time was 2 hours in this Example. After Nocodazole treatment, the culture solution was collected. By centrifugation at 1000rpm for 5 minutes, cells in metaphase were collected. Since cells in metaphase have low adhesiveness and float free in the culture solution, the cells in metaphase can be collected by obtaining the culture solution. Cells in metaphase exhibit a round form, having a diameter approximately 1.5 times larger than cells in the interphase of the cell cycle. The ratio of cells in metaphase among collected cells was examined by their forms. The result showed that approximately 80% of the cells were cells in metaphase.

hCG was administered to a B6CBF1 female mouse, and unfertilized ova in the second meiotic metaphase were collected after 14 hours. An approximately 1/4 incision was made in the zona pellucida of this unfertilized ovum with a microtool, glass knife. Next, enucleation of the unfertilized ovum was performed in an M2 solution containing cytochalasin B (5µg/ml), by inserting a microtool glass pipette in the incision in the zona pellucida, removing the somewhat transparent portion of the cytoplasm of the unfertilized ovum, and confirming the presence of chromosomes in the ovum. It should be noted that these microscopic operations were performed under an inverted microscope fitted with micromanipulators, and the following operations were also performed with this device. A first nucleus transplantation was performed using the unfertilized ovum that was enucleated in this manner as recipient cytoplasm, and the nucleus of the above-described embryonic stem cell as a donor nucleus. The nucleus transplantation was performed in a ova working solution (a solution with M2 medium as a basic medium) containing cytochalasin B (5µg/ml) and Nocodazole (0.4µg/ml), under a microscope fitted with a Hoffman modulation contrast device. By using such a microscope, the state of chromosome condensation of the cells infused in the pipette can be observed. And cells in metaphase can be certainly selected. Fusion of the donor nucleus with the recipient cytoplasm was conducted using deactivated Sendai virus. The rate of fusion was about 75%. After completion of fusion, culture was performed for 1 to 2 hours in a culture solution, and then for 4 to 6 hours in a culture solution comprising 10mM strontium to activate the nuclei. Results showed that 93% of the transplanted nuclei had discharged second polar bodies and had begun normal development. Some of the ova that were processed in this manner were cultured *in vitro* for 4 days in microdroplets of CZB culture solution. On day four of *in vitro* culture, ova which had developed into morula or blastocysts were transplanted into the uterus of a surrogate mother mouse on the 3rd day of pseudopregnancy.

Remaining ova were used for a second nucleus transplantation. The second nucleus transplantation was performed using a nucleus of an established ovum at 8 to 12 hours after activation processing as a donor nucleus, and an enucleated fertilized ovum (F1 x F1) at 8 to 12 hours after insemination as a recipient cytoplasm. Enucleation and transplantation operations were performed similarly to those described above. Further, post-nucleus transplantation operations were performed similarly to those described above.

The state of development for each transplantation operation is shown in Table 1.

**Table 1**

| Transplantation Method | Number of cultured embryos | Number of Transplantable embryos (%) | Number of Implantations (%) | Number of neonates (%) | Number of viable individuals (%) |
|---|---|---|---|---|---|
| 1 nucleus transplantation | 548 | 280(51.1) | 133(24.3) | 20(3.7) | 17(85.0) |
| 2 nucleus transplantations | 269 | 183(68.0) | 103(38.3) | 7(2.6) | 6(85.7) |

As shown in Table 1, 51.1 % of ova obtained by a single nuclear transplantation operation were developed into transplantable embryos, and finally 3.7% (20 individuals) developed into neonates. 68.0% of ova obtained by 2 transplantation operations were developed into transplantable embryos, and 2.6% (7 individuals) developed into neonates. Of these neonates, 17 and 6 individuals exhibited normal growth, respectively.

It should be noted that operations similar to those described above were performed using fibroblasts in place of embryonic stem cells but only 2 of the 5 neonates exhibited normal growth.

### Effect of the Invention

The present invention provides a novel method of creating a genetically engineered animal and cloned animal.

In the method of creating a genetically engineered animal according to the present invention, all created individuals are genetically engineered individuals since the desired genetically engineered cell can be selected at the cell culture stage, and genetically engineered animals can be obtained efficiently. Further, with this method, unlike conventional methods using embryonic stem cells, it is possible to directly obtain a genetically engineered animal without obtaining a germ-line chimera individual. Further, by performing genetic engineering on the cultured cells of the line that is sought to be created, there is no need for backcrossing to the line of interest. Therefore, with this method, a genetically engineered animal can be created in a short period.

Furthermore, cloned animals can be obtained efficiently since the mortality rate during the developmental stage is low for cloned animals created by the method of creating a cloned animal according to the present invention.

## Claims

1. A method of creating a genetically engineered animal which comprises the following steps:
(1) a step of genetically engineering animal cells;
(2) a step of synchronizing the cell cycle of the genetically engineered animal cells to metaphase;
(3) a step of transplanting the nucleus of a cell whose cell cycle was synchronized to metaphase into cytoplasm of-an enucleated unfertilized ovum;
(4) a step of subjecting the transplanted nucleus to activation processing; and
(5) a step of obtaining a neonate derived from the transplanted nucleus.

2. A method of creating a genetically engineered animal which comprises the following steps:
(1) a step of genetically engineering animal cells;
(2) a step of synchronizing the cell cycle of the genetically engineered animal cells to metaphase;
(3) a step of transplanting the nucleus of the cell whose cell cycle was synchronized to metaphase, into cytoplasm of an enucleated unfertilized ovum;
(4) a step of subjecting the transplanted nucleus to activation processing;
(5) a step of transplanting the nucleus that was subjected to activation processing to an enucleated fertilized ovum; and
(6) a step of obtaining a neonate derived from the transplanted nucleus.

3. The method of creating a genetically engineered animal according to claim 1 or 2, wherein the animal cell is an embryonic stem cell.

4. A method of creating a cloned animal which comprises the following steps:
(1) a step of synchronizing the cell cycle of embryonic stem cells to metaphase;
(2) a step of transplanting the nucleus of a cell whose cell cycle was synchronized to metaphase into cytoplasm of an enucleated unfertilized ovum;
(3) a step of subjecting the transplanted nucleus to activation processing; and
(4) a step of obtaining a neonate derived from the transplanted nucleus.

5. A method of creating a cloned animal which comprises the following steps:
(1) a step of synchronizing the cell cycle of embryonic stem cells to metaphase;
(2) a step of transplanting the nucleus of a cell whose cell cycle was synchronized to metaphase into cytoplasm of an enucleated unfertilized ovum;
(3) a step of subjecting the transplanted nucleus to activation processing;
(4) a step of transplanting the nucleus that was subjected to activation processing to an enucleated fertilized ovum; and
(5) a step of obtaining a neonate derived from the transplanted nucleus.
